# EUROPEAN PATENT APPLICATION

(11) **EP 4 324 462 A1**
(43) Date of publication of application: **21.02.2024**
(21) Application number: 22788188.5
(22) Date of filing: 13.04.2022
(51) Int. Cl.: A61K 31/198, A23L 33/105, A61P 3/02, A61P 13/12, A61P 17/00, A61P 25/18, A61P 43/00

(54) **SIRTUIN OR KLOTHO ACTIVATOR OR EXPRESSION ENHANCER, NAD+ INCREASING AGENT, AND SENOLYTIC AGENT**

(30) Priority: 13.04.2021 JP 2021067931
(71) Applicant: Wakunaga Pharmaceutical Co., Ltd., Osaka-shi Osaka 532-0003 (JP)
(72) Inventor: SUZUKI, Junichiro, Akitakata-shi, Hiroshima 739-1195 (JP); FUJII, Kayo, Akitakata-shi, Hiroshima 739-1195 (JP); TAKASHIMA, Miyuki, Akitakata-shi, Hiroshima 739-1195 (JP); MIKI, Satomi, Akitakata-shi, Hiroshima 739-1195 (JP); USHIJIMA, Mitsuyasu, Akitakata-shi, Hiroshima 739-1195 (JP); MATSUTOMO, Toshiaki, Akitakata-shi, Hiroshima 739-1195 (JP)
(74) Representative: Wächtershäuser & Hartz Patentanwaltspartnerschaft mbB
(86) International application number: PCT/JP2022/017713
(87) International publication number: WO 2022/220265

(57) **Abstract**

Provided is a new use of S-1-propenylcysteine, a salt thereof, or a composition containing the same.

A sirtuin or Klotho activator or expression enhancer containing S-1-propenylcysteine or a salt thereof as an active ingredient.

## Description

### Technical Field

The present invention relates to an agent used for activating or enhancing expression of sirtuins or Klotho, increasing NAD⁺, and suppressing senescent cells.

### Background Art

Sirtuins and Klotho are known as molecules, for example, in nematodes, flies, and mammals, whose high expression can prolong lifespan while deficiency thereof can shorten such lifespan. Genes of sirtuins that are NAD⁺ dependent deacetylases are widely conserved from bacteria to eukaryotes. Sirtuins have been attracting attention as candidates for longevity genes in animals, because deficiency of Sir2, which is a sirtuin homolog of yeast and nematodes, shortens the lifespan, while overexpression of Sir2 prolongs the lifespan. There are seven sirtuins (SIRT1 to SIRT7) in mammals, and among them, SIRT1 having a structure and function that is the most similar to yeast Sir2 has been revealed to be probably involved in controlling a wide range of cellular functions such as gene expression associated with aging, intracellular metabolism, energy consumption, inflammation, and stress response pathways (Non-Patent Literature 1).

In recent years, it has been revealed that sirtuins may be related to specific diseases. For example, Non-Patent Literature 2 shows that cognitive functions including immediate memory, classical conditioning, and spatial learning are impaired in SIRT1 knockout mice. Conversely, overexpression of SIRT1 is observed to exhibit ordered synaptic plasticity and memory. It has been revealed that SIRT1 acts on normal learning, memory, and synaptic plasticity.

Nicotinamide mononucleotide (NAD⁺) functions as a coenzyme for dehydrogenases and a substrate for sirtuins in living bodies. It is also known that an increase in NAD⁺ amount enhances sirtuins activity. Synthesis of NAD is controlled by nicotinamide phosphoribosyltransferase (NAMPT). It is known that decreased function of NAMPT with age leads to reduced NAD⁺ amount and decreased sirtuins activity. Non-Patent Literature 3 discloses that administration of nicotinamide riboside, a precursor of NAD⁺, suppresses the senescent cells in the brain.

In addition, Klotho maintains phosphorus homeostasis as a receptor for fibroblast growth factor 23. Non-Patent Literature 4 discloses that since the expression level of Klotho is significantly correlated with the onset, progression and complications of chronic kidney disease (CKD), Klotho can be potentially used, for example, as a biomarker for diagnosis and prevention of CKD.

Cellular senescence is a phenomenon of the occurrence of irreversible cell cycle arrest that results from excessive DNA damage via accumulation of DNA replication errors associated with cell division, oxidative stress, radiation, activation of oncogenes, or the like while activating p16/RB pathways and p53/p21 pathways, and inducing inhibitors of cyclin inhibitor kinase. Cells that have undergone the cellular senescence (the senescent cells) accumulate in tissues due to accelerated cellular senescence and reduced removal ability caused by decline in mitochondria and immune functions associated with aging. The senescent cells accumulated in tissues secrete, for example, inflammatory cytokines and proteases, which are called cellular senescence associated secretory factors, thereby damaging surrounding tissues and accelerating aging of tissues and living bodies. On the other hand, it has been suggested that removal of senescent cells should prevent or delay tissue dysfunction and suppress the senescence (Non-Patent Literature 5). In addition, it has been revealed that an increase in senescent cells is involved in kidney injuries (Non-Patent Literature 6).

On the other hand, S-1-propenylcysteine is a cysteine derivative represented by the following Formula (1), and is one of sulfur-containing components that can be found in plants of genus Allium such as garlic.

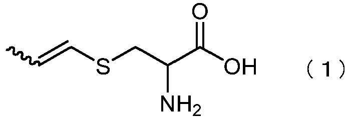

It has been reported that S-1-propenylcysteine has pharmacological actions such as immune function regulating action (Patent Literature 1), antihypertensive action (Patent Literature 2), antioxidant action (Patent Literature 3), blood flow improving action (Patent Literature 4), autophagy activation action (Patent Literature 5), and preventing, treating, or ameliorating actions on periodontal disease (Patent Literature 6). However, nothing is known about S-1-propenylcysteine's activation of sirtuins and Klotho and suppression of senescent cells.

### Citation List

### Patent Literature

Patent Literature 1: WO 2016/088892 A
Patent Literature 2: WO 2016/199885 A
Patent Literature 3: JP 2020-029529 A
Patent Literature 4: WO 2019/031442 A
Patent Literature 5: WO 2019/235597 A
Patent Literature 6: WO 2020/230813 A

### Non-Patent Literature

Non-Patent Literature 1: Trends Cell Biol. 2014;24(8):464-71.
Non-Patent Literature 2: J Neurosci 2010;30(29):9695-9707.
Non-Patent Literature 3: Cell Metab. 2018 Mar 6;27(3):513-528.
Non-Patent Literature 4: BMC Nephrol. 2018;19:285.
Non-Patent Literature 5: Nature. 2011 Nov 2;479(7372):232-6.
Non-Patent Literature 6: Front Pharmacol. 2019;10:770.

### Summary of Invention

### Technical Problem

The present invention relates to providing a new use of S-1-propenylcysteine, a salt thereof, or a composition containing the same.

### Solution to Problem

The present inventors made various studies on usefulness of S-1-propenylcysteine or a salt thereof, and found that S-1-propenylcysteine or a salt thereof are excellent in activating sirtuins or Klotho and suppressing senescent cells which express p16, p21, and p53 genes or proteins, thereby completing the present invention.

In other words, the present invention relates to the following 1) to 26) .
1) A sirtuin or Klotho activator or expression enhancer containing S-1-propenylcysteine or a salt thereof as an active ingredient.
2) An agent containing S-1-propenylcysteine or a salt thereof that is administered to an animal in need thereof and is used for sirtuin or Klotho activation or expression enhancement.
3) An NAD⁺ increasing agent containing S-1-propenylcysteine or a salt thereof as an active ingredient.
4) An NAD⁺ increasing agent containing S-1-propenylcysteine or a salt thereof that is administered to an animal in need thereof.
5) A senolytic agent containing S-1-propenylcysteine or a salt thereof as an active ingredient.
6) A senolytic agent for non-cancer cells containing S-1-propenylcysteine or a salt thereof that is administered to an animal in need thereof.
7) The senolytic agent according to 5) or 6) which suppresses cells expressing p16, p21 or p53 genes.
8) The agent according to any one of 1) to 7) for acting on one or more organs selected from the group consisting of kidney and brain.
9) The agent according to any one of 1) to 8) being in a form of a food additive or food.
10) The agent according to any one of 1) to 9) for use in preventing, treating or ameliorating symptoms resulting from low activity or low expression of sirtuins or Klotho or symptoms resulting from accumulation of senescent cells.
11) The agent according to any one of 1) to 10) for use in preventing, treating or ameliorating symptoms or diseases selected from kidney injuries and cognitive/memory disorders.
12) A use of S-1-propenylcysteine or a salt thereof for producing a sirtuin or Klotho activator or expression enhancer.
13) A use of S-1-propenylcysteine or a salt thereof for producing an agent that is administered to an animal in need thereof and is used for sirtuin or Klotho activation or expression enhancement.
14) A use of S-1-propenylcysteine or a salt thereof for producing an NAD⁺ increasing agent.
15) A use of S-1-propenylcysteine or a salt thereof for producing an NAD⁺ increasing agent that is administered to an animal in need thereof.
16) A use of S-1-propenylcysteine or a salt thereof for producing a senolytic agent.
17) A use of S-1-propenylcysteine or a salt thereof for producing a senolytic agent for non-cancer cells that is administered to an animal in need thereof.
18) S-1-propenylcysteine or a salt thereof for use in sirtuin or Klotho activation or expression enhancement.
19) S-1-propenylcysteine or a salt thereof that is administered to an animal in need thereof and is used for sirtuin or Klotho activation or expression enhancement.
20) S-1-propenylcysteine or a salt thereof that is used for increasing NAD⁺.
21) S-1-propenylcysteine or a salt thereof that is administered to an animal in need thereof and is used for increasing NAD⁺.
22) S-1-propenylcysteine or a salt thereof that is used for suppressing senescent cells.
23) S-1-propenylcysteine or a salt thereof that is administered to an animal in need thereof and is used for suppressing senescent cells of non-cancer cells.
24) A method for activating or enhancing expression of sirtuins or Klotho by administering S-1-propenylcysteine or a salt thereof to an animal in need thereof.
25) A method for increasing NAD⁺ by administering S-1-propenylcysteine or a salt thereof to an animal in need thereof.
26) A method for suppressing senescent cells of non-cancer cells by administering S-1-propenylcysteine or a salt thereof to an animal in need thereof.

### Advantageous Effects of Invention

The present invention provides a new use of S-1-propenylcysteine, a salt thereof, or a composition containing the same.

### Brief Description of Drawings

Fig. 1 illustrates a sirtuin activation action in cerebral cortex by administrating S-1-propenylcysteine. **: There is a significant difference at 1% level compared to a control group.
Fig. 2 illustrates a change in a SIRT1 protein mass in the hippocampus by administrating S-1-propenylcysteine. *: There is a significant difference at 5% level compared to a control group.
Fig. 3 illustrates changes in NAD⁺ amount in the hippocampus by administrating S-1-propenylcysteine. **: There is a significant difference at 1% level compared to a control group.
Fig. 4 illustrates a change in p53 protein that is a senescent cell marker in the hippocampus by administrating S-1-propenylcysteine. **: There is a significant difference at 1% level compared to a control group.
Fig. 5 illustrates changes in expression levels of SIRT1 and Klotho genes in a kidney by administrating S-1-propenylcysteine. *: There is a significant difference at 5% level compared to a control group.
Fig. 6 illustrates changes in the expression levels of p16 and p21 genes that are senescent cell markers in a kidney by administrating S-1-propenylcysteine. *: There is a significant difference at 5% level compared to a control group.
Fig. 7 illustrates changes in KIM-1 and NGAL gene protein masses that are markers of kidney injuries in a kidney by administrating S-1-propenylcysteine. *: There is a significant difference at 5% level compared to a control group, and **: there is a significant difference at 1% level compared to the control group.
Fig. 8 illustrates cognition/memory retention by administrating S-1-propenylcysteine. **: There is a significant difference at 1% level compared to a control group.

### Description of Embodiments

"S-1-propenylcysteine" has a cis or a trans configuration as indicated by a wavy line in Formula (1) below. In the present invention, it is preferable that S-1-propenylcysteine has a large ratio of trans isomer, and when the total of the trans isomer and cis isomer is taken as 100%, a ratio of the trans isomer is more preferably 50 to 100%, more preferably 75 to 100%, more preferably 80 to 100%, and still more preferably 90 to 100%.

In addition, existence of an asymmetric carbon in a cysteine structure leads to existence of an optical isomer. However, it may be any of a D isomer, an L isomer, or a racemic form.

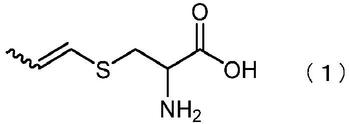

A salt of S-1-propenylcysteine is a physiologically acceptable salt, and may be either an acid addition salt or a base addition salt. Examples of the acid addition salt include (a) salts with mineral acids such as hydrochloric acid, sulfuric acid, nitric acid, or phosphoric acid, (b) salts with organic carboxylic acids such as formic acid, acetic acid, citric acid, fumaric acid, gluconic acid, malic acid, succinic acid, tartaric acid, trichloroacetic acid, or trifluoroacetic acid, and (c) salts with sulfonic acids such as methanesulfonic acid, benzenesulfonic acid, p-toluenesulfonic acid, mesitylenesulfonic acid, or naphthalenesulfonic acid; and examples of the base addition salt include (a) salts with alkali metals such as sodium or potassium, (b) salts with alkaline earth metals such as calcium or magnesium, (c) ammonium salts, and (d) salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine.

Furthermore, S-1-propenylcysteine or a salt thereof can exist not only in an unsolvated form but also as a hydrate or a solvate, and such hydrate or solvate can be any crystal form depending on production conditions. Therefore, a sulfur-containing compound or a salt thereof in the present invention includes all stereoisomers, hydrates, solvates, and all polymorphic crystalline or amorphous forms.

S-1-propenylcysteine or a salt thereof can be obtained by an organic synthesis method ([1] Tetrahedron Letter, 1975, 37, 3201-3202; [2] Synthesis, 2006, 20, 3367-3369; [3] Bull. Korean Chem. Soc. 2011, 32(1), 319-320).

In addition, S-1-propenylcysteine or a salt thereof can be obtained by processing a plant of genus Allium by various methods. Examples include a method of obtaining S-1-propenylcysteine by reacting a heat-treated plant of genus Allium with an enzyme containing protease or lactase derived from Bacillus subtilis (JP 6105871 B2), a method of obtaining S-1-propenylcysteine by coexisting squeezed juice or extract of the plant of genus Allium with an enzyme having a gamma-glutamyl transpeptidase or glutaminase activity (JP 2015-144571 A), a method of obtaining S-1-propenylcysteine in which garlic is inoculated with yeast and fermented (KP 2010-072874-5), and a method in which the plant of genus Allium is aged in an aqueous ethanol solution for one month or more to obtain S-1-propenylcysteine (WO 2016/088892 A, Molecules. 2017; 22: 570) .

Here, examples of the plant of genus Allium include garlic (Allium sativum L.), onion (Allium cepa L.), elephant garlic (Allium ampeloprasum L.), Allium tuberosum, and green onion (Allium fistulosum L.). Such plants may be used solely or in combination. In addition, as the plant of genus Allium, a raw plant can be used as it is, or if necessary, be removed of its outer shell, cut or shredded, or made into powder or extract using a solvent capable of producing a medicine or food. Examples of the solvent include water and alcohol, and a solvent obtained by adding an acid or a basic substance to the solvent.

S-1-propenylcysteine or a salt thereof used in the present invention can be either an isolated and purified product, a crude product, the above plant or a processed product thereof, or a fraction in which a content of S-1-propenylcysteine or a salt thereof is increased by an extraction operation from the above plant. For example, as shown below, it can be obtained by 1) extracting a plant of genus Allium in a 10 to 50% aqueous ethanol solution at 0 to 80°C for one month or more (step 1), and 2) collecting an ethanol-eluted fraction after a solid-liquid separation of the obtained extract (step 2).

The aqueous ethanol solution used in step 1) can be a 10 to 50% aqueous ethanol solution. However, the solution is preferably prepared to have an ethanol concentration of 20 to 40%. In addition, a treatment temperature can be set within a range of 0 to 80°C, preferably 10 to 60°C, and more preferably 20 to 40°C. A treatment period before extraction can be at least one month or more under the above conditions, preferably 1 to 20 months, and more preferably 1 to 10 months. In addition, this step can be performed in an airtight, sealed or tight container considering for example, hygiene and ethanol volatilization. However, it is preferable to use a tight container.

In step 2), the extract obtained in step 1) is subjected to the solid-liquid separation to collect the ethanol-eluted fraction. By appropriately concentrating the collected material, it is possible to obtain an extracted fraction containing the sulfur-containing compound or a salt thereof. The extracted fraction can be used as it is, or be used after being appropriately dried by spray-drying or the like.

Furthermore, an isolation of S-1-propenylcysteine or a salt thereof from the extracted fraction containing S-1-propenylcysteine or a salt thereof can be realized by dialysis using a dialysis membrane with a molecular exclusion size of 3000 to 4000 as necessary, and then appropriately combining the dialysis with an adsorption/separation method using a cation exchange resin or a means of separation and purification by normal phase chromatography or reversed-phase chromatography.

Here, examples of the adsorption/separation method using the cation exchange resin include adsorbing on the cation exchange resin (for example, Amberlite (manufactured by the Dow Chemical Company), DOWEX (manufactured by the Dow Chemical Company), DIAION (manufactured by Mitsubishi Chemical Corporation)) and eluting with 0.1 to 3 N ammonia water.

Examples of the normal phase chromatography include a method of eluting with a chloroform/methanol/water mixture or the like using a silica gel column.

Examples of the reversed-phase chromatography include a method of eluting with a 0.01 to 3% aqueous formic acid solution or the like using an octadecylsilyl column.

Preferably, a method is mentioned, wherein the ethanol-extracted fraction is dialyzed (the dialysis membrane: the molecular exclusion size 3000 to 4000), then adsorbed on a cation exchange resin, then eluted with 0.5 to 2 N aqueous ammonia, and the eluate is subjected to silica gel column chromatography (the solvent: chloroform/methanol/water mixture) to collect a fraction containing a target product, and further subjected to preparative reversed-phase column chromatography (the solvent: 0.1 to 0.5% aqueous formic acid solution) to collect the target product.

The ratio of the trans isomer of S-1-propenylcysteine obtained in the above steps 1) and 2) is approximately 70 to 90% when the total of the trans isomer and the cis isomer is taken as 100%.

S-1-propenylcysteine or a salt thereof in the present invention is generally of low toxicity, because, for example, LD₅₀ value of dilute ethanolic extract of garlic (an extract content: 14.5%, an alcohol number: 1.18), which is one of the raw materials, is 50 ml/kg or more in any of oral, intraperitoneal, and subcutaneous administration routes (The Journal of Toxicological Sciences. 1984; 9: 57.), and plants of genus Allium such as garlic and onion are commonly used as food.

As shown in test examples to be described later, S-1-propenylcysteine or a salt thereof increased the SIRT1 activity amount in the cerebral cortex of senescence-accelerated mice. In addition, repeated administration for two weeks increased SIRT1 expression in the hippocampus and the kidney. In addition, NAD⁺ (having a function known to improve functions of sirtuins) actually increased in the animal's brain. In addition, repeated administration for two weeks increased Klotho expression in the kidney. Therefore, S-1-propenylcysteine, a salt thereof, or a composition containing the same may be a sirtuin or Klotho activator or expression enhancer.

In addition, as shown in the test examples to be described later, S-1-propenylcysteine or a salt thereof suppressed cells expressing p16 and p21 in the kidney and p53 in the hippocampus, which are markers of senescent cells. Therefore, S-1-propenylcysteine, a salt thereof, or a composition containing the same can be a senolytic agent.

Considering the common technical knowledge that activation or expression enhancement of sirtuins and Klotho, senescent cell suppression, and NAD⁺ increasing have a mechanism common to each other, S-1-propenylcysteine, a salt thereof, or a composition containing the same can be used as a sirtuin or Klotho activator or expression enhancer, an NAD⁺ increasing agent, or the senolytic agent to act in the kidney and the brain.

The sirtuin or Klotho activator or expression enhancer and the senolytic agent of the present invention may be used for preventing, treating or ameliorating symptoms resulting from low activity or low expression of sirtuins or Klotho, or symptoms resulting from an increase in senescent cells.

These symptoms are not limited and may be due to kidney injuries or cognitive/memory disorders. Examples of the kidney injuries include diseases in which KIM-1 and NGAL are highly expressed, acute kidney injury, diabetic nephropathy, and nephrotic syndrome. Examples of the cognitive/memory disorders include neurological disorders in the cerebral cortex or the hippocampus, learning disorders, and memory disorders (including short-term and long-term).

As described above, functional deterioration of SIRT1 or Klotho and accelerated cellular senescence are considered to be related to symptoms and diseases such as kidney injuries and cognitive/memory disorders (Non-Patent Literatures 2, 4, and 6). Therefore, the sirtuin or Klotho activator or expression enhancer and senolytic agent of the present invention may be used for preventing, treating or ameliorating symptoms or diseases such as kidney injuries and cognitive/memory disorders (including short-term and long-term).

In the present invention, "sirtuins" mean sirtuin proteins and homologs thereof. Humans are known to have 1 to 7 sirtuins (SIRT1 to SIRT7). However, SIRT1 is preferred in the present invention.

"Klotho" means Klotho proteins and homologs thereof. Human Klotho is known to have α, β, and γ-Klotho. However, α-Klotho is preferable in the present invention.

In the present invention, "sirtuin or Klotho activation" includes, for example, increasing the sirtuin or Klotho activity amount.

"Sirtuin or Klotho expression enhancement" means an increase in a transcript of a sirtuin or Klotho gene, and/or a sirtuin or Klotho protein, and includes, for example, activating transcription and/or translation of a gene, improving stability of the transcript and/or the protein, inhibiting degradation of the transcript and/or proteolysis, etc.

In the present invention, the "cellular senescence" refers to a phenomenon in which the irreversible cell cycle arrest occurs in a cell. The cellular senescence has been observed, for example, in brain cells (such as hippocampal cells and cerebral cortex cells) and kidney cells. The "senescent cell suppression" refers to one or both of removal of senescent cells and prevention or delay of a cellular senescence phenomenon.

The p16, p21, and p53 genes are known as molecular markers of DNA damage that causes the cellular senescence. However, the senolytic agent of the present invention more preferably suppresses mitochondrial dysfunction that accelerates the cellular senescence and DNA damage. In addition, the senescent cells can be more effectively suppressed by enhancing the immune functions.

The sirtuin or Klotho activator and the senolytic agent of the present invention may be in the form of medicine or food, or in a form of a material or a preparation added thereto.

In addition, the food includes a food product, a functional food product, a food product labeled with functionality, a food product for patients, a food product for specified health uses, and a nutritional supplement (a supplement), which are based on the concept of sirtuin or Klotho activation or senescent cell suppression and where an action based on the function is described and displayed as necessary.

A dosage form of S-1-propenylcysteine medicine is preferably a dosage form suitable for oral administration. As a specific dosage form of an oral administration preparation, for example, a solid preparation may be in a form of a tablet, a capsule, a fine particle, a pill, or a granule, and a liquid preparation may be in a form of an emulsion, a solution, a suspension, or a syrup. Such a pharmaceutical preparation can be prepared by appropriately blending, for example, an excipient, a binder, a disintegrant, a lubricant, a colorant, a flavoring agent, and a pH adjuster, into the sulfur-containing compound or a salt thereof of the present invention as necessary, and preparing the pharmaceutical preparation according to a conventional method.

However, the dosage form of the medicine is not particularly limited, and may be a dosage form suitable for parenteral administration, for example, a dosage form suitable for intravenous (an injection and catheter), transmucosal (a liquid or ointment), or intracranial administration (a catheter).

A form of S-1-propenylcysteine food is not particularly limited, and can take various forms such as a solid food product, a semi-fluid food product, a gel-like food product, a tablet, a caplet, and a capsule, and more specifically a form of various food products such as a confection, a beverage, a seasoning, a processed fishery product, a processed meat food product, bread, and a health food product.

Such food can be produced via a conventional method by appropriately blending a food material used usually for producing such food with the sulfur-containing compound or a salt thereof of the present invention.

The medicine or food can contain other substances involved in sirtuin or Klotho activation, expression enhancement and senescent cell suppression, for example, resveratrol or nicotinamide mononucleotide. In addition, for example, vitamins, lipids, and minerals that alleviate inflammation, such as vitamin C, vitamin E, vitamin B2, vitamin B6, niacin, hesperidin, α-lipoic acid, glutathione, coenzyme Q10, zinc, magnesium, and omega-3 fatty acid can be contained.

A preferred intake amount of the aforementioned medicine or food per day varies depending on factors, for example, subjects to be ingested, forms of ingestion, types of materials, additives to be ingested simultaneously, and ingestion intervals. However, it is preferable to ingest 0.001 to 10 mg/kg of S-1-propenylcysteine or a salt thereof per day, and it is more preferable to ingest 0.1 to 1 mg/kg per day. In addition, a daily dose can be optionally divided into 2 to 4 times for ingestion.

Examples of the subject to be administered or ingested include a living organism with reduced activity amount or expression of sirtuin or Klotho or increased senescent cells. However, the examples may also be a healthy living organism without these problems. The living organism is not limited to the animals described so far, and includes plants, fungi (including yeast), nematodes, and cells (may be derived from the above animals or plants), and the living organism is preferably an animal. Examples of the animals include vertebrates (preferably rodents and primates), fish, birds, insects, and reptiles, and in particular, rodents (particularly rats and mice) and primates (particularly humans and monkeys) are preferable.

As far as the present inventors have known, there is no proven fact that administrating a processed product of the plant of genus Allium or the sulfur-containing component contained in the plant of genus Allium to an animal leads to sirtuin activation, Klotho activation or expression enhancement, increased NAD⁺ in an animal body, and no proven fact demonstrating senescence suppression of non-cancer cells in the animal body. These were unexpectedly discovered as a result of intensive studies by the present inventors.

Examples include humans suffering from kidney injuries and cognitive/memory disorders, and animals (for example, humans) hoping to prevent the disease. However, healthy living organisms are also preferable.

Plant sirtuins have been suggested to be involved in protection from genomic instability and cellular oxidative damage, gamete formation, fruit development and maturation, leaf senescence, and regulation of photosynthetic activity (Front Plant Sci. 2018 Jul 5; 9: 961.).

Examples of the plant are not particularly limited and may include eggplants such as tomatoes, green peppers, chili peppers, and eggplants; cucurbits such as cucumbers, squashes, melons, and watermelons; fresh vegetables and condiment vegetables such as celery, parsley and lettuce; Allium vegetables such as green onions, onions, and garlic; beans such as soybeans, peanuts, green beans, peas, and azuki beans; other fruit vegetables such as strawberries; taproots such as radishes, turnips, carrots, and burdock; potatoes such as taros, cassava, potatoes, sweet potatoes and Chinese yams; soft vegetables such as asparagus, spinach and Japanese honewort; flowering plants such as lisianthus, stock flowers, carnations, and chrysanthemums; grains such as rice and corn; lawn grass such as bent grass and Manila grass; oil crops such as rapeseed and sunflower; sugar crops such as sugarcanes and sugar beet; fiber crops such as cotton and ragweed; forage crops such as clover, sorghum, and dent corn; deciduous fruit trees such as apples, pears, grapes, and peaches; citrus fruits such as Citrus unshiu, lemons, and grapefruits, and woody trees such as satsuki, azaleas, and cedars.

Examples of the dosage form to be administered to the plants include powders, granules, granular agents, wettable powders, flowables, emulsions, and pastes. Such dosage may be administered directly to plants, or may be administered indirectly via soil, a hydroponic solution, or a culture medium. Specific examples include a soil treatment agent, a foliage treatment agent, a seed treatment agent before seeding, a treatment agent for a plant before transplantation, a treatment agent for a plant during transplantation, a hydroponic solution, and a culture medium.

### Examples

### Preparation Example 1 Ethanol-extracted fraction of garlic

About 1 kg of peeled garlic bulbs and about 1000 mL of 30% ethanol were put in a container and sealed. The container was left at room temperature for 1 to 10 months, and stirred as appropriate. Solid and liquid were separated from the mixture, and the liquid was dried by spray-drying to obtain a yellow-brown powder.

Preparation Example 2 Separation of S-1-propenylcysteine from the ethanol-extracted fraction of garlic
(1) The ethanol-extracted fraction of garlic obtained in Preparation Example 1 was put into a dialysis tube with a pore size of 3500, and dialyzed against purified water. The dialysate was passed through a cation exchange resin Dowex 50Wx8 (H+), and the resin was thoroughly washed with purified water. Amino acids adsorbed to the resin were eluted with 2 N ammonia and concentrated under reduced pressure. A concentrate was applied to a silica gel column, and subjected to column chromatography using a chloroform/methanol/water mixture as a solvent. The fraction containing the target product (S-1-propenylcysteine) were collected and concentrated. The concentrate was dissolved in water, and chromatography was performed using a preparative reversed-phase column (the octadecylsilyl column) with 0.1% formic acid as a solvent to collect the target product, and the solvent was removed by freeze drying. The obtained lyophilizate was confirmed to be a mixture of trans-S-1-propenylcysteine and cis-S-1-propenylcysteine (trans isomer: cis isomer = 8: 2) by comparing the structure with spectra obtained from the following standard substances using NMR (solvent: heavy water) and a mass spectrometer.

### Trans-S-1-propenylcysteine

¹H-NMR (500 MHz, in D₂O-NaOD, δ): 1.76 (d, 3H, J = 7.0 Hz), 2.98 (dd, 1H, J = 7.5, 14.5 Hz), 3.14 (dd, 1H, J = 4.5, 14.5 Hz) 3.69 (dd, 1H, J = 4.5, 7.5 Hz), 5.10-5.14 (m, 1H), 6.02 (d, 1H, J = 15.5 Hz);
¹³C-NMR (125 MHz, in D₂O-NaOD, δ): 17.61, 33.53, 53.70, 119.92, 132.12, 172.73,
HRMS: observed [M+H]⁺ = 162.0583, calculated [M+H] ⁺ = 162.0581

### Cis-S-1-propenylcysteine

¹H-NMR (500 MHz, in D₂O, δ): 1.74 (d, 3H, J = 7.0 Hz), 3.21 (dd, 1H, J = 7.5, 15.0 Hz), 3.31 (dd, 1H, J = 4.5, 15.0 Hz), 3.95 (dd, 1H, J = 4.5, 7.5 Hz), 5.82-5.86 (m, 1H), 6.01 (d, 1H, J = 9.5 Hz);
¹³C-NMR (125 MHz, in D₂O-NaOD, δ): 13.89, 33.88, 54.16, 122.58, 127.78, 172.63.
HRMS: observed [M+H] ⁺ = 162.0580, calculated [M+H] ⁺ = 162.0581

### (2) Measurement of S-1-propenylcysteine in the ethanol-extracted fraction of garlic

500 mg to 1 g of the ethanol-extracted fraction of garlic obtained in Preparation Example 1 (1) was put in a container, and 20 mM hydrochloric acid solution of S-n-3 butenyl cysteine was added thereto as an internal standard, and the volume was adjusted to 20 mL with 20 mM hydrochloric acid. After the mixture was well stirred, a part thereof was taken and centrifuged at 1750 G for about 10 minutes. A part of the supernatant obtained was taken and subjected to centrifugal filtration using a centrifugal filtration unit (Amicon Ultra, cutoff: 3000) (15000 rpm, 10 min). 20 µL of the obtained filtered product was taken and derivatized using an AccQ-Tag Derivatization Kit (Waters). Separately, a standard compound was dissolved in 20 mM hydrochloric acid, and the same procedure as for a sample was performed to prepare a standard solution for calibration curve. A sample solution and the standard solution were chromatographed on an Acquity UPLC system (Waters) to determine the content. As a result, S-1-propenylcysteine was of 3.7 ± 0.3 mg/g dry matter.

### Test Examples

### Sirtuin or Klotho activation action

### (1) Sample preparation

Preparation of a test liquid for evaluating biological activity was performed as follows. When evaluating the biological activity, all test liquids were prepared at the time of use.
(a) About 5 mg of S-1-propenylcysteine (cis/trans mixture) produced in Preparation Example 2 was weighed precisely, and dissolved in 10 mL of purified water to prepare an administration liquid.
(b) About 6 mg of S-1-propenylcysteine (cis/trans mixture) produced in Preparation Example 2 was weighed precisely and dissolved in 1 mL of culture liquid. The liquid was used as a stock solution, diluted appropriately, and subjected to an in vitro test.

### (2) Animals for evaluation tests

Animals for evaluating the biological activity were bred as follows. Senescence-accelerated mice SAMP8 (male) for testing were purchased from Japan SLC, Inc. After purchase, the animals were acclimatized for one week and used as animals for the evaluation tests.

### (3) Preparation of human neuroblasts for evaluation tests

Human neuroblast line SH-SY5Y cells were cultured in Dulbecco's Modified Eagle's Medium added with 10% fetal bovine serum, antibiotic penicillin, and streptomycin solution, and were used as cells for the evaluation tests.

### (4) Sirtuin activation test

A SIRT1_GLO kit manufactured by Promega Corporation was purchased to measure the sirtuins activity. A lysate of the cerebral cortex was placed in a 96-well plate, various reaction reagents were added, and then luminous intensity of luciferase was measured by a plate reader.

### (a) SIRT1 activation action (in vivo):

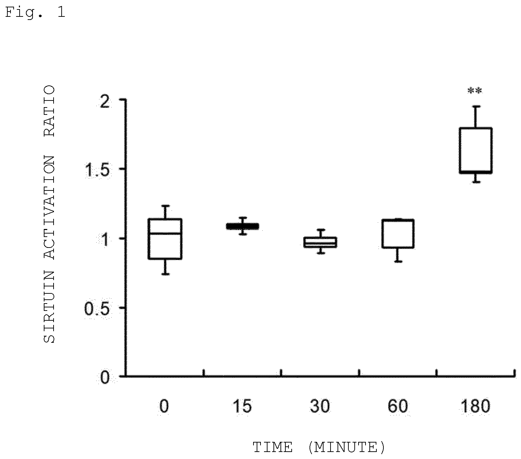

A single dose of the sample prepared in the above (1)(a) was orally administrated to animals for the evaluation tests in the above (2), and then cerebral cortices of such animals were extracted 15, 30, 60, and 180 minutes later. The cerebral cortices were crushed using an automatic tissue crusher and lysed by adding a cell dissolution liquid. Centrifugation was performed and the sirtuins activity in the supernatant thereof was measured. Results are shown in Fig. 1.

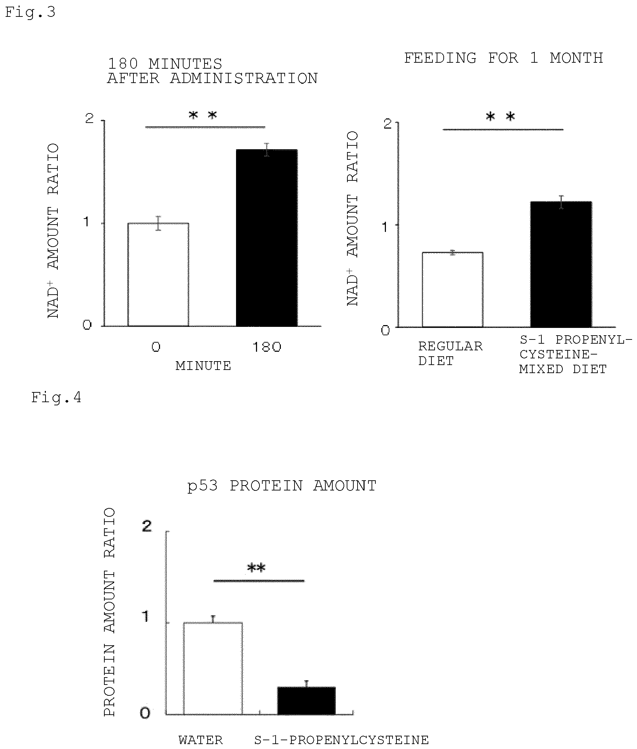

Sirtuin activity increased 180 minutes after administrating S-1-propenylcysteine. (Fig. 3)

### (b) SIRT1 protein increasing action (in vivo):

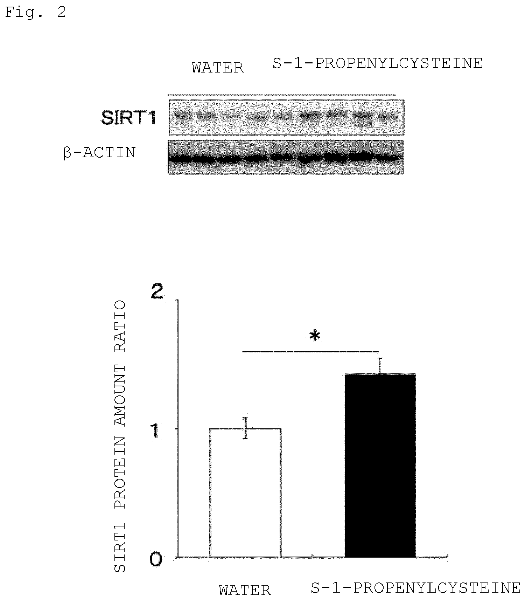

After repeated oral administration of the sample prepared in the above (1)(a) to animals for the evaluation tests in the above (2) for two weeks, hippocampi of such animals were excised. After the hippocampi were crushed using an automatic tissue crusher, the cells were lysed with a RIPA cell lysis buffer manufactured by Millipore Corporation which was diluted 10-fold with purified water added with combined protease/phosphatase inhibitors manufactured by Thermo Fisher Scientific K.K., and then centrifuged (10,000 rpm, 10 min, 4°C), and the supernatant was used as a cell extract. The cell extract was used for analysis by Western Blotting according to a conventional method. An anti-SIRT1 antibody (manufactured by Biolegend) and an anti-β-actin antibody (manufactured by Fujifilm Wako Pure Chemical Corporation) were used as antibodies. Results are shown in Fig. 2. S-1-propenylcysteine increased the SIRT1 protein mass.

### (C) NAD⁺ amount increasing action (in vivo):

A single dose of the sample prepared in (1)(a) was orally administered to animals for the evaluation tests in the above (2), and then cerebral cortices of such animals were excised 180 minutes later. In addition, the sample prepared in the above (1)(a) was mixed with food and fed to animals for the evaluation tests in the above (2) for one month, and then cerebral cortices of such animals were excised. The NAD⁺ amount in the cerebral cortices was measured using NAD⁺/NADH Assay Kit manufactured by Dojindo Laboratories. Results are shown in Fig. 3. S-1-propenylcysteine increased NAD⁺ amount.

### (5) Suppression action on expression of cellular senescence marker p53 protein in the hippocampus (in vivo):

After repeated oral administration of the sample prepared in the above (1)(a) to animals for the evaluation tests in the above (2) for six weeks, hippocampi of such animals were excised. After the hippocampi were crushed using an automatic tissue crusher, the cells were lysed with a RIPA cell lysis buffer manufactured by Millipore Corporation which was diluted 10-fold with purified water added with a protease inhibitor and a phosphatase inhibitor manufactured by Roche, and then centrifuged (10,000 rpm, 10 min, 4°C), and the supernatant was used as a cell extract. The cell extract was used for analysis by Western Blotting according to a conventional method. An anti-p53 antibody (manufactured by Proteintech Group, Inc.) and an anti-β-actin antibody (manufactured by Medical and Biological Laboratories Co., Ltd.) were used as antibodies. Results are shown in Fig. 4. S-1-propenylcysteine reduced a p53 protein mass.

### (6) Enhancement action of SIRT1 and Klotho gene expression in the kidney (in vivo):

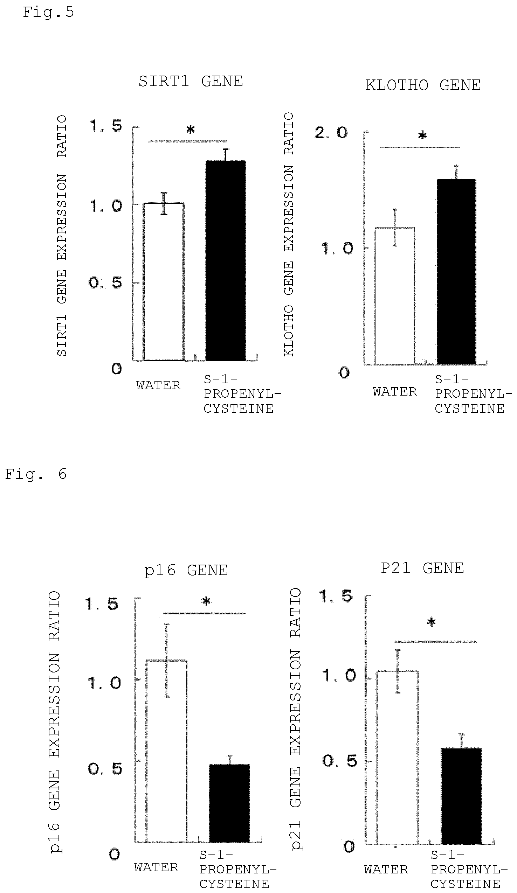

After repeated oral administration of the sample prepared in the above (1)(a) to the animals for the evaluation tests in the above (2) for two weeks, kidneys of such animals were excised. The kidneys were crushed using an automatic tissue crusher, and then RNA was extracted using a TRIzol solution manufactured by Thermo Fisher Scientific K.K. cDNA was synthesized using PRIMEScript RT reagent Kit with gEraser manufactured by Takara. The synthesized cDNA was subjected to real-time PCR using KAPA SYBR Fast qPCR kit manufactured by Nippon Genetics Co., Ltd. Results are shown in Fig. 5. S-1-propenylcysteine increased the expression of SIRT1 and Klotho genes in the mouse kidney.

### (7) Suppression action on expression of senescent cell markers p16 and p21 genes in the kidney (in vivo):

After repeated oral administration of the sample prepared in the above (1)(a) to the animals for the evaluation tests in the above (2) for two weeks, kidneys of such animals were excised. The kidneys were crushed using an automatic tissue crusher, and then RNA was extracted using a TRIzol solution manufactured by Thermo Fisher Scientific K.K.

cDNA was synthesized using PRIMEScript RT reagent Kit with gEraser manufactured by Takara. The synthesized cDNA was subjected to real-time PCR using KAPA SYBR Fast qPCR kit manufactured by Nippon Genetics Co., Ltd. Results are shown in Fig. 6. S-1-propenylcysteine reduced cells expressing p16 and p21 genes in the kidneys of senescence-accelerated mice.

### (8) Inhibitory action on kidney injuries (in vivo)

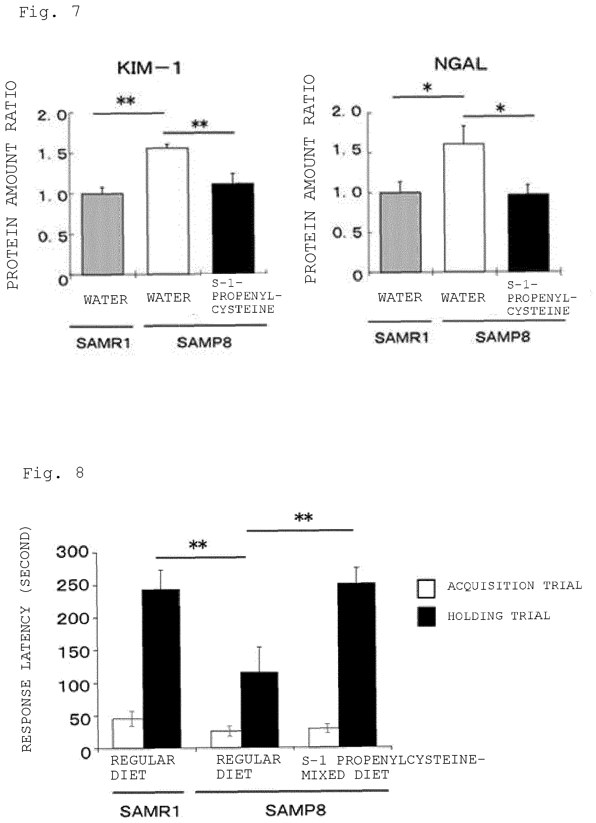

After repeated oral administration of the sample prepared in the above (1)(a) to the animals for the evaluation tests in the above (2) for two weeks, kidneys of such animals were excised. After the kidneys were crushed using an automatic tissue crusher, the cells were lysed with an RIPA cell lysis buffer manufactured by Millipore Corporation which was diluted 10-fold with purified water added with a protease inhibitor and a phosphatase inhibitor manufactured by Roche and then centrifuged (10,000 rpm, 10 min, 4°C), and the supernatant was used as a cell extract. The cell extract was used for analysis by Western Blotting according to a conventional method. A kidney injury molecule (KIM-1) antibody (manufactured by R&D System) and a lipocalin-2 (NGAL) antibody (manufactured by Proteintech Group, Inc.), KIM-1 and NGAL being markers of kidney injuries, and an anti-GAPDH antibody (manufactured by Fujifilm Wako Pure Chemical Corporation) were used as antibodies. Results are shown in Fig. 7. S-1-propenylcysteine reduced KIM-1 and NGAL.

### (9) Cognitive/memory retention action (in vivo):

The sample prepared in the above (1)(a) was mixed with food and fed to animals for evaluation test in the above (2) for four months, and then a passive avoidance test was performed. The passive avoidance test is a test utilizing a habit that a mouse prefers a dark place, and on Day 1, a mouse already in a bright room was moved to a dark room while receiving an electrical shock (50 V/1 second), and the time it took for the mouse to enter the dark room was taken as response latency of an acquisition trial. After two months, the mouse was put in the bright room again and the time it took to move to the dark room (the response latency of a holding trial) was used as an index of memory. Results are shown in Fig. 8. S-1-propenylcysteine ameliorated aging-related decline in cognitive and memory functions.

## Claims

1. A sirtuin or Klotho activator or expression enhancer containing S-1-propenylcysteine or a salt thereof as an active ingredient.

2. An agent containing S-1-propenylcysteine or a salt thereof that is administered to an animal in need thereof and is used for sirtuin or Klotho activation or expression enhancement.

3. An NAD⁺ increasing agent containing S-1-propenylcysteine or a salt thereof as an active ingredient.

4. An NAD⁺ increasing agent containing S-1-propenylcysteine or a salt thereof that is administered to an animal in need thereof.

5. A senolytic agent containing S-1-propenylcysteine or a salt thereof as an active ingredient.

6. A senolytic agent for non-cancer cells containing S-1-propenylcysteine or a salt thereof that is administered to an animal in need thereof.

7. The senolytic agent according to claim 5 or 6, which suppresses cells expressing p16, p21 or p53 genes.

8. The agent according to any one of claims 1 to 7 for acting on one or more organs selected from the group consisting of kidney and brain.

9. The agent of any one of claims 1 to 8 being in a form of a food additive or food.

10. The agent according to any one of claims 1 to 9 for use in preventing, treating or ameliorating symptoms that result from low activity or low expression of sirtuins or Klotho or symptoms that result from accumulation of senescent cells.

11. The agent according to any one of claims 1 to 10 for use in preventing, treating or ameliorating symptoms or diseases selected from kidney injuries and cognitive/memory disorders.

12. A use of S-1-propenylcysteine or a salt thereof for producing a sirtuin or a Klotho activator or expression enhancer.

13. A use of S-1-propenylcysteine or a salt thereof for producing an agent that is administered to an animal in need thereof and is used for sirtuin or Klotho activation or expression enhancement.

14. A use of S-1-propenylcysteine or a salt thereof for producing an NAD⁺ increasing agent.

15. A use of S-1-propenylcysteine or a salt thereof for producing an NAD⁺ increasing agent that is administered to an animal in need thereof.

16. A use of S-1-propenylcysteine or a salt thereof for producing a senolytic agent.

17. A use of S-1-propenylcysteine or a salt thereof for producing a senolytic agent for non-cancer cells that is administered to an animal in need thereof.

18. S-1-propenylcysteine or a salt thereof for use in sirtuin or Klotho activation or expression enhancement.

19. S-1-propenylcysteine or a salt thereof that is administered to an animal and used for sirtuin or Klotho activation or expression enhancement.

20. S-1-propenylcysteine or a salt thereof for use in increasing NAD⁺.

21. S-1-propenylcysteine or a salt thereof that is administered to an animal in need thereof and is used for increasing NAD⁺.

22. S-1-propenylcysteine or a salt thereof for use in suppressing senescent cells.

23. S-1-propenylcysteine or a salt thereof that is administered to an animal in need thereof and is used for suppressing senescent cells of non-cancer cells.

24. A method for activating or enhancing expression of sirtuins or Klotho by administering S-1-propenylcysteine or a salt thereof to an animal in need thereof.

25. A method for increasing NAD⁺ by administering S-1-propenylcysteine or a salt thereof to an animal in need thereof.

26. A method for suppressing senescent cells of non-cancer cells by administering S-1-propenylcysteine or a salt thereof to an animal in need thereof.
